# EUROPÄISCHE PATENTSCHRIFT

(11) **EP 1 378 147 B1**
(45) Veröffentlichungstag und Bekanntmachung des Hinweises auf die Patenterteilung: **29.09.2004**
(21) Anmeldenummer: 02732603.2
(22) Anmeldetag: 11.04.2002
(51) Int. Cl.: H05B 41/00

(54) **VERFAHREN UND VORRICHTUNG ZUM BETREIBEN EINES ELEKTRISCHEN GASENTLADUNGSMODULS**
METHOD AND DEVICE FOR OPERATING AN ELECTRIC GAS DISCHARGE MODULE
PROCEDE ET DISPOSITIF DE FONCTIONNEMENT D'UN MODULE ELECTRIQUE A DECHARGE GAZEUSE

(30) Priorität: 11.04.2001 DE 10118078
(43) Veröffentlichungstag der Anmeldung: 07.01.2004
(73) Patentinhaber: ITVI Schweiz AG, 9471 Buchs SG 2 (CH)
(72) Erfinder: RUMP, Hanns, 63840 Hausen (DE); KIESEWETTER, Olaf, 98716 Geschwenda (DE); GERHART, Jessica, 59427 Unna (DE)
(74) Vertreter: Mierswa, Klaus
(86) Internationale Anmeldenummer: PCT/EP2002/004047
(87) Internationale Veröffentlichungsnummer: WO 2002/085077

(56) Entgegenhaltungen:
- DE-A- 10 013 841
- US-A- 6 019 949
- PATENT ABSTRACTS OF JAPAN vol. 1999, no. 03, 31. März 1999 (1999-03-31) -& JP 10 325560 A (CORONA CORP), 8. Dezember 1998 (1998-12-08)

## Beschreibung

### Technisches Gebiet

Die Erfindung betrifft ein Verfahren zum Betreiben eines elektrischen Gasentladungsmoduls, insbesondere für dielektrisch behinderte Entladung, wobei das Gasentladungsmodul imstande ist, Ionen und Ozon in der Luft zu erzeugen und zwei Elektroden aufweist, zwischen denen eine Wechsel-Hochspannung aus einem Hochspannungserzeuger angelegt und somit ein elektrisches Feld erzeugt wird, gemäß dem Oberbegriff des Anspruchs 1 sowie eine Vorrichtung zum Betreiben eines derartigen elektrischen Gasentladungsmoduls gemäß dem Oberbegriff des Anspruchs 12.

### Stand der Technik:

Es besteht vielfach der Wunsch, in Belüftungsanlagen von Gebäuden, Fahrzeugen, Klimageräten und Kraftfahrzeugen als auch in kompakten Luftaufbereitungs- und Luftkonditionierungsanlagen die Luft nicht nur von Stäuben und Partikeln, sondern auch von Gerüchen und vor allem von krankmachenden Keimen zu befreien. Die hauptsächlich im Betrieb feuchte Oberfläche des Verdampfers in Fahrzeugen mit Klimaanlagen ist ein Nährboden für Keime und Pilze aller Art. Die vom Luftstrom abgerissenen und in die Kabine bzw. Räume getragenen Bakterien, Pilze und Keime sowie die Stoffwechselprodukte derselben sondern einen üblen Geruch ab und sind gesundheitlich äußerst bedenklich.

In den Patentanmeldungen US-A-6,019,949, DE 195 43 296.7, DE 196 46 269.1, DE 196 51 403.7, DE 199 02 304.2, DE 19919 623.0, DE 199 31366.0, DE 199 33 180.4, DE 1 00 14 485.3, DE 1 00 13 841.1, DE 1 00 04 326.7, DE 1 00 58 476.4, PCT/DE 00/02164, PCT/EP 01/00672 und DE 10 103 905.0 sind verschiedene Methoden vorgeschlagen worden, um diesen Problemen unter Einsatz von Ionisations- und Ozonisierungsapparaten abzuhelfen.

In der Patentanmeldung DE 199 31 366.0 ist z.B. eine flache Baugruppe mit planarem Aufbau vorgeschlagen worden, die vorteilhaft und langzeitstabil Ozon und Sauerstoffionen nach dem physikalischen Prinzip der dielektrisch behinderten Entladung nach Anlegen einer elektrischen Hochfrequenz-Hochspannung erzeugt.

In der Patentanmeldung DE 1 00 13 841.1 ist des Weiteren ein Verfahren vorgeschlagen worden, um die Ozon- und Ionenproduktion mit Hilfe einer elektrischen Regelschaltung konstant zu halten. Die Ozon- und Ionenproduktion unterliegt verschiedensten Einflüssen, wobei sich bei Langzeitbetrieb die elektrischen und mechanischen Werte der zur Ozon- und Ionenproduktion eingesetzten Baugruppen verändern. Das wirkt sich vorteilhaft auf die Ozon- und Ionenproduktion auf, so daß bei konventionellen Anlagen keine konstanten Bedingungen erreicht werden, was bei der bekannten Toxizität von Ozon bedenklich ist.

Aus der Patentanmeldung DE 1 00 58 476.4 ist bekannt, die Produktionsmenge von Ozon ― und Sauerstoffionen als Funktion der Luftmenge zu begreifen und mit Signalen des die Ventilatoren steuernden zentralen Lüftungssteuergerät so auf den Ozon- und Ionenerzeuger einzuwirken, daß auch bei unterschiedlichen Luftmengen eine konstante Konzentration an Ozon und Ionen entsteht. Dies ist die Voraussetzung für eine kontrollierte und sichere Funktion der Geräte, weil bei zu geringer Konzentration keine wesentliche Wirkung mehr besteht und weil bei zu hoher Ozonkonzentration negative Effekte auf das beteiligte Material erfolgen, ohne daß die lufttechnische Wirkung größer wird.

In der Patentanmeldung DE 196 51 403.7 ist vorgeschlagen, einen Ozon- und Ionenerzeuger in Strömungsrichtung vor dem Verdampfer in Klimaanlagen von z.B. Kraftfahrzeugen, Gebäuden oder kompakten Klimatisierungsanlagen eine Ozon- und Ionenquelle anzuordnen und den feuchten Verdampfer mit Ozon- und Luftionen zu umströmen. Vor allem Ozon löst sich im Wasser auf der Oberfläche des Verdampfers und bildet Hydroradikale, die extrem bakterizid und fungizid wirken und erprobt und sicher jegliche biologische Aktivität auf der Oberfläche des Verdampfers unterbinden. In Strömungsrichtung nach dem Verdampfer wird ein Katalysator vorgeschlagen, der überschüssigen Ozon zu normalem zweiatomigem Sauerstoff abbaut.

Das Verständnis dieser Vorgänge ist von Bedeutung, weil Wasser sich auf allen Oberflächen und in allen Materialien befindet und dieses Wasser mit zugeführtem Ozon Hydroradikale bildet, welche extrem wirksam gegen Bakterien, Viren und Pilzen als auch Gerüchen sind. In den vorgenannten Patentanmeldungen wird die Wirkung vor allem auf die oxidative chemische Wirkung des dreiatomigen und einatomigen Ozon abgestellt.

Aufgrund der Notwendigkeit, die Ozonkonzentration in der ausgeblasenen Luft auf einen von der amerikanischen Umweltbehörte ( US-EPA / Environment Protection Agency ) und der DIN / EN-Norm EN 60335-2-65 (1995) zwingend geforderten Wert sicher so zu begrenzen, daß niemals in den so belüfteten Räumen ein Ozon-Pegel von mehr als 50ppb entstehen kann, ist es ein Zweck der Erfindung, die nützliche Wirkung ionisierter Luftionen mit der technischen und sicheren Anwendung aktiver Sauerstoffionen unter Einhaltung der geforderten Grenzwerte zu verbinden.

Bei Anwendungen in Apparaten zur Aufbereitung von Atemluft sind folgende Eigenschaften wünschenswert:
- Sicherer Betrieb, niemals darf die Konzentration von Ozon Werte erreichen, die bedenklich sind. Allerdings ist die Beimischung geringer Mengen Ozon in die Luft erwünscht, weil die Natur in gesunder Außenluft stets auch geringe Mengen Ozon kennt, was sich nützlich auf die Luftqualität auswirkt. Die Ozonkonzentration muß unterhalb der Geruchsschwelle ( ca. 30-40ppb ) und unter dem bereits erwähnten Grenzwert der US-EPA von 50ppb sein.
- Kontrollierte Erzeugung großer Mengen negativer und positiver Sauerstoffionen.
- Langzeitstabilität und automatische Konstanthaltung der Produktionswerte.
- Steuerbar nach externen Einflüssen, wie zum Beispiel Luftmenge, Luftqualität.
- Die Ionen- und Ozonerzeuger müssen die einmal gewählte Produktionsmenge auch langfristig und unter Einfluß von z.B. Feuchte konstant zu halten.

Wird in bestimmten Anwendungen gefordert, mehrheitlich aktive zweiatomige Sauerstoffionen zu produzieren und Ozon kontrolliert nur in sehr kleinen Mengen zu erzeugen, erfolgt dies dem Stand der Technik entsprechend nach dem Verfahren der Spitzenionisation, Koronar-Prinzip. Allerdings kann mit dieser Technik weder die Langzeitstabilität als auch die Mengen an Ionen als auch die Anteile von Ozon zugesichert werden.

### Technische Aufgabe:

Der Erfindung liegt die Aufgabe zu Grunde, ein Verfahren und eine Vorrichtung zum Betreiben eines elektrischen Gasentladungsmoduls zu schaffen, welche eine automatische Beschränkung der Ozonproduktion des Gasentladungsmoduls ermöglichen und die Gefahr einer Fortsetzung des Betriebes des Gasentladungsmoduls im Falle einer Fehlfunktion wesentlich vermindert.

Diese Aufgabe wird erfindungsgemäß bei einem Verfahren der eingangs genannten Gattung dadurch gelöst, dass ein bestimmter Schwellenwert der Wechsel-Hochspannung gegeben ist, oberhalb desselben mittels des Gasentladungsmoduls elektrische Entladeimpulse, welche ihrerseits Ozon produzieren, und Ionen erzeugt werden, wobei durch die Entladeimpulse elektrische Signale hervorgerufen werden, welche zum Zwecke der automatischen Beschränkung der Ozonproduktion mittels einer Rückkopplung dazu herangezogen werden, die Wechsel-Hochspannung unter den Schwellenwert auf einen solchen Wert abzusenken, daß keine Entladeimpulse und somit kein Ozon mehr entstehen, jedoch weiterhin Ionen in dem elektrischen Feld erzeugt werden.

Die Aufgabe wird ferner bei einer Vorrichtung der eingangs genannten Gattung dadurch gelöst, dass daß ein bestimmter Schwellenwert der Wechsel-Hochspannung gegeben ist, oberhalb desselben durch das Gasentladungsmodul elektrische Entladeimpulse, welche ihrerseits Ozon produzieren, und Ionen erzeugt werden, wobei durch die Entladeimpulse elektrische Signale hervorgerufen werden, welche zum Zwecke der automatischen Beschränkung der Ozonproduktion dazu dienen, mittels einer Rückkopplung die Wechsel-Hochspannung unter den Schwellenwert auf einen solchen Wert abzusenken, daß keine Entladeimpulse und somit kein Ozon mehr entstehen, jedoch weiterhin Ionen in dem elektrischen Feld erzeugt werden.

Gemäß einer bevorzugten Variante wird die Wechsel-Hochspannung nach der Absenkung unter den Schwellenwert wieder so weit über den Schwellenwert erhöht, daß erneut Entladeimpulse und somit Ozon erzeugt werden, welche wiederum dazu herangezogen werden, die Wechsel-Hochspannung wiederum unter den Schwellenwert abzusenken und so fort, so dass aufgrund der Rückkopplung die Wechsel-Hochspannung um den Schwellenwert pendelt.

Zu diesem Zweck kann die Vorrichtung so eingerichtet sein, dass die Wechsel-Hochspannung nach der Absenkung unter den Schwellenwert wieder so weit über den Schwellenwert steigt, daß erneut Entladeimpulse und somit Ozon erzeugt werden, welche die Wechsel-Hochspannung wiederum unter den Schwellenwert absenken und so fort, so dass aufgrund der Rückkopplung die Wechsel-Hochspannung um den Schwellenwert pendelt.

Vorteilhafterweise gewährleistet die Erfindung insbesondere, daß die automatische Beschränkung der Ozonproduktion unverändert auch dann weiterhin gegeben bleibt, wenn sich der Schwellenwert z.B. aufgrund von Elektroden-Abbrand, Temperaturschwankungen oder Änderungen der relativen Luftfeuchte verschiebt, da sich die pendelnde Wechsel-Hochspannung automatisch an den verschobenen Schwellenwert anpaßt. Durch eine solche Verschiebung verändert sich daher insbesondere die mittlere, geringe Ozonproduktionsrate nicht.

Die Grundfrequenz der Wechsel-Hochspannung kann z.B. zwischen 20 und 100KHz betragen. Die einzelnen Entladeimpulse sind in der Regel von weit kürzerer Zeitdauer als die Halbwellen der Wechsel-Hochspannung; sie können z.B. 0,1 Mikrosekunden andauern und dabei Amplituden von z.B. 50 Volt erreichen; diese Werte hängen selbstverständlich von den Betriebsbedingungen und der Geometrie das Gasentladungsmoduls ab.

Die Erfindung besitzt den Vorteil, dass mit derselben konstanten Bedingungen hinsichtlich der Ozon- und Ionenproduktion erreicht werden, weshalb die Lehre der Erfindung allgemein bei Luftaufbereitungsanlagen vorteilhaft einsetzbar ist, weil die Oxidationskraft und damit die Bakterizidität des in Hydroradikalen abgespalteten einatomigen Sauerstoffes, O₁, sehr viel höher ist als die Oxidationskraft von Ozon, O₃.

Die Erfindung nutzt des Weiteren in vorteilhafter Weise die Erkenntnis, daß Ionisations- und Ozonisierungsapparate nach dem Prinzip der dielektrisch behinderten Entladung stabil und automatisch geregelt mit einer sich selbst einstellenden elektrischen Wechsel-Hochspannung betrieben werden, in welchem die Luft in hohem Maße ionisiert, Ozon jedoch in nur geringem Maße erzeugt wird. Dabei nutzt die Erfindung die Tatsache, daß zur Ionisation lediglich sehr hohe elektrische Felder aufgebaut werden und keine hohen Ströme fließen, allerdings zur Ozonisierung kurze Entladungsimpulse hoher kinetischer Energie benötigt werden. Die erfindungsgemäße Rückkopplung bzw. Spannungsregelung hält die Betriebsspannung der Ionisatoren stets und automatisch in einem Bereich, nämlich in der Grenze zum Beginn der Ozonisierung. Insbesondere macht die Erfindung die Handhabung von Ozonisierungseinrichtungen sicherer und berechenbarer.

Gemäß einer Variante nimmt die durch einen Hochspannungserzeuger mit einem Steuereingang erzeugte Wechsel-Hochspannung bei Anlegen einer anwachsenden Spannung an den Steuereingang ab und umgekehrt, wobei aus den elektrischen Signalen eine Regelspannung gewonnen wird, welche an den Steuereingang rückgekoppelt wird. Regelspannung und Wechsel-Hochspasnnung verhalten sich somit gegenläufig. Zur Gewinnung der Regelspannung aus den Signalen können dieselben von der Hochspannung abgekoppelt, gleichgerichtet und geglättet werden. Gegebenenfalls können die Signale nach der Glättung zusätzlich verstärkt und integriert werden. Die Absenkung oder die Erhöhung der Wechsel-Hochspannung kann z.B. treppenfunktionsartig erfolgen.

Gemäß einer bevorzugten Variante erfolgt die Absenkung der Hochspannung mit einer betragsmäßig höheren zeitlichen Änderungsrate als die Erhöhung der Wechsel-Hochspannung, so daß sich ein jeweils sägezahnähnlicher zeitlicher Verlauf der Regelspannung sowie der Hochspannung ergibt.

Die Wechsel-Hochspannung kann um einen vorgegebenen Verringerungsbetrag abgesenkt oder um einen vorgegebenen Erhöhungsbetrag erhöht werden. D.h. die Absenkung kann so vorgenommen werden, daß die Wechsel-Hochspannung um den vorgegebenen Verringerungsbetrag verringert wird. Auf diese Weise wird eine bestimmte Verringerung der Wechsel-Hochspannung erzwungen. Ebenso kann die Erhöhung der Wechsel-Hochspannung so vorgenommen werden, daß diese um den vorgegebenen Erhöhungsbetrag gesteigert und somit eine bestimmte Steigerung der Wechsel-Hochspannung erzwungen wird. Diese Vorgehensweise ist insbesondere dann vorteilhaft, wenn der Schwellenwert z.B. aufgrund von Abnutzung der Elektroden, Temperaturänderungen, Luftfeuchteschwankungen usw. driftet.

Gemäß einer anderen Variante wird die Wechsel-Hochspannung auf eine vorgegebene, unterhalb der Schwellenspannung liegende Minimalspannung abgesenkt oder auf eine vorgegebene, oberhalb der Schwellenspannung liegende Maximalspannung erhöht. D.h. die Absenkung kann so vorgenommen werden, daß die Wechsel-Hochspannung im Gegensatz zu der letztgenannten Variante nicht um einen bestimmten Betrag, sondern auf eine bestimmte Spannung verringert wird. Ebenso kann die Erhöhung der Wechsel-Hochspannung so vorgenommen werden, daß diese nicht um einen bestimmten Betrag, sondern auf eine bestimmte Spannung gesteigert wird.

Gemäß einer bevorzugten Ausführungsform der erfindungsgemäßen Vorrichtung weist der Signalweg der Rückkopplung nach dem Gasentladungsmodul einen Hochfrequenzübertrager, einen Demodulator, einen Verstärker mit einem Eingang sowie einen Integrierer auf, wobei der Hochspannungserzeuger eine Ansteuerungsschaltung umfaßt, wobei
- die Ansteuerungsschaltung einen Steuereingang aufweist und die Wechsel Hochspannung so zu steuern imstande ist, daß diese
   - konstant bleibt, falls am Steuereingang eine konstante Spannung anliegt,
   - ansteigt, falls am Steuereingang eine abnehmende Spannung anliegt, und
   - absinkt, falls am Steuereingang eine zunehmende Spannung anliegt, so daß sich die Größe der Wechsel-Hochspannung gegenläufig zur Größe der am Steuereingang anliegenden Spannung verhält,
- die Primärwicklung des Hochfrequenzübertragers mit dem Gasentladungsmodul in Serie geschaltet ist und die elektrischen Signale induktiv auf die Sekundärwicklung überträgt, welche die Signale an den Demodulator abgibt und welche einseitig an Masse gelegt ist,
- der Demodulator die Signale gleichrichtet und glättet und danach als Eingangssignal an den Eingang des Verstärkers abgibt,
- der Verstärker das Eingangssignal verstärkt und als Ausgangssignal an den Integrierer abgibt, und
- der Integrierer das Ausgangssignal mit einer vorgegebenen Zeitkonstanten zu einem Regelsignal integriert und dieses dem Steuereingang aufgibt.

Der Hochfrequenzübertrager kann so ausgelegt sein, daß er die Grundfrequenz der Wechsel-Hochspannung im wesentlichen nicht überträgt, so daß er die Signale im wesentlichen von der Wechsel-Hochspannung abtrennt. Der Demodulator kann eine erste Diode, einen ersten Kondensator und einen ersten Widerstand umfassen, wobei die erste Diode zum Zweck der Gleichrichtung der elektrischen Signale zwischen den Hochfrequenz-Übertrager und den Verstärker zwischengeschaltet ist, und der Eingang des Verstärkers über den ersten Kondensator und parallel hierzu über den ersten Widerstand an Masse gelegt ist. Der Verstärker kann ein Impedanzwandler sein. Der Integrierer kann eine zweite Diode, einen zweiten Kondensator und einen zweiten Widerstand umfassen, wobei die zweite Diode zwischen den Verstärker und den Steuereingang zwischengeschaltet ist, und der Steuereingang über den zweiten Kondensator und parallel hierzu über den zweiten Widerstand an Masse gelegt ist.

Gemäß einer bevorzugten Ausführungsform der Erfindung weist der aus dem Hochspannungserzeuger, dem Gasentladungsmodul, dem Hochfrequenzübertrager, dem Demodulator, dem Verstärker und dem Integrierer gebildete Regelkreis eine große Regelungssteilheit und hohe Hysterese auf, wobei die Zeitkonstante für das Ansprechen der Regelschleife kleiner ist als die Zeitkonstante für das Abklingen der Regelspannung mit dem Ergebnis, daß die Regelspannung eine Sägezahnfunktion bildet.

Die Absenkung und die Erhöhung der Wechsel-Hochspannung erfolgen gemäß einer Variante der Erfindung mittels eines Mikroprozessors, an welchen die Regelspannung angelegt wird. Zu diesem Zweck kann die Vorrichtung einen Mikroprozessor aufweisen, an welchen die Regelspannung angelegt ist und welcher die Wechsel-Hochspannung erhöht und absenkt.

Gemäß einer weiteren Variante des Verfahrens wird die Wechsel-Hochspannung durch eine Überwachungsschaltung, welche das zeitliche Verhalten der Regelspannung überwacht, dann abgeschaltet, wenn die Dynamik der Regelspannung für mindestens eine vorgegebene Kontrollzeit eine vorgegebene Mindest-Dynamik nicht erreicht. Die Überwachungsschaltung ist bevorzugt Bestandteil der erfindungsgemäßen Vorrichtung. Gemäß einer Ausführungsform umfaßt die erfindungsgemäße Vorrichtung daher eine Überwachungsschaltung, welche das zeitliche Verhalten der Regelspannung überwacht und die Wechsel-Hochspannung dann abschaltet, wenn die Dynamik der Regelspannung für mindestens eine vorgegebene Kontrollzeit eine vorgegebene Mindest-Dynamik nicht erreicht.

Die Erfindung überprüft damit vorteilhaft in einem unabhängigen und übergeordneten Schaltkreis die bei ordnungsgemäßer Funktion der Regelung erzeugten Signale und schaltet den Ionisator automatisch dann ab, wenn dieses Signal nicht mehr vorhanden ist, so dass die Funktion des Hochspannungserzeugers in übergeordneter Funktion unterbrochen wird.

Gemäß einer weiteren Variante des Verfahrens wird die um einen bestimmten Wert pendelnde Regelspannung über eine Leitung als Triggerimpulse auf eine elektrische Auswerteschaltung gegeben, welche nach dem Prinzip eines monostabilen Multivibrators arbeitet und einen Ausgang aufweist, wobei mit Beginn des Verfahrens der Ausgang der Auswerteschaltung durch einen Startimpuls so gesetzt wird, daß dessen auf ein Schaltgerät wirkender Ausgang die Versorgungsspannung des Hochspannungserzeugers einschaltet, wobei, falls über die Leitung keine Triggerimpulse den zuerst erreichten Zustand aufrechterhalten, die Auswerteschaltung automatisch in den anderen logischen Zustand fällt, und dadurch das Schaltgerät die Funktion des Hochspannungserzeugers in übergeordneter Funktion unterbricht.

Die Erfindung stellt somit eine Verfahren zum Betrieb von Ionisations- und Ozonisierungseinrichtungen zum Zwecke der Behandlung von Atemluft oder der Sterilisierung vorzugsweise feuchter Oberflächen oder Textilien oder medizinischer Gerätschaften unter Verwendung elektrischer Ionisatoren und Ozonerzeuger, vorzugsweise solcher nach dem Prinzip der dielektrisch behinderten Entladung arbeitender Apparate, welche elektronisch durch Rückkopplung und Ausnutzung der Entladungsimpulse geregelt werden, zur Verfügung, bei welchem die Ionisations-und Ozonisierungsapparate automatisch geregelt in einem definiertem Spannungsbereich betrieben werden, in welchem vorzugsweise Ionisationseffekte und in geringerem Maße Ozonisationseffekte auftreten. Die bei Auftreten der Entladungsimpulse zur Regelspannungsgewinnung demodulierten Impulse stellen die Regelspannung in sehr kurzer Zeit auf einen Wert ein, der die Hochspannung auf einen Wert absenkt, in welchem keine Entladungen mehr stattfinden, elektrischen Felder dagegen weiterhin Ionen produzieren. Aus den elektrischen Entladungen, welche für die Ozonproduktion verantwortlich sind, kann eine Regelspannung gebildet werden, die einem Regelkreis mit großer Regelungssteilheit und hoher Hysterese zugeführt wird, wobei die Zeitkonstante für das Ansprechen der Regelschleife kleiner ist als die Zeitkonstante für das Abklingen der Regelspannung mit dem Ergebnis, daß die Regelspannung eine Sägezahlfunktion bildet.

Des Weiteren ist es vorzugsweise möglich, aus dem zwischen Minimal-und Maximalwert oszillierendem Regelspannungssignal ein digitales Signal abzuleiten, welches eine zu Beginn des Betriebes gesetzte monostabiles Schaltgruppe nachtriggert, wobei bei Ausbleiben der Nachtriggerung die monostabile Baugruppe in den Normalzustand fällt und damit einen Schaltkreis sperrt, welcher die Spannungsversorgung der Hochspannungserzeugung sichert. Aus den Regelspannungsänderungen lassen sich entsprechende Triggerimpulse gewinnen. Die Regelung kann volldigital von einem Mikroprozessor ausgeführt werden.

Die Hochspannung kann über eine Treppenfunktion so lange erhöht. werden, bis Entladungsimpulsen in der Rückkopplungsschleife registriert werden mit der Folge, daß dann die Hochspannung einen definierten Betrag herabgesetzt wird und im Anschluß sich wieder treppenfunktionsmäßíg erhöht.

Des weiteren kann die Absenkung der Hochspannung nach Erreichen der Entladungszone treppenfunktionsartig auf einen Wert erfolgen, bei dem keine Entladungsimpulse mehr auftreten. Nach Erreichen dieser Zone kann die Hochspannung wieder treppenfunktionsartig erhöht werden.

Die Erfindung beschreibt eine Vorrichtung und ein Verfahren der eingangs genannten Gattungen, mit welchen vorteilhaft stabile Arbeitspunkte erreicht werden und die aufgrund von Langzeitkonstanz für Anwendungen zur Atemluftaufbereitung geeignet sind.

Kurzbeschreibung der Zeichnung, in welcher zeigen:
- Fig. 1: verschiedene Bereiche der an einem Gasentladungsmodul anliegenden Wechsel-Hochspannung,
- Fig.2: einen schematischen Schaltplan einer Ausführungsform einer erfindungsgemäßen Vorrichtung,
- Fig.3: einen sich gemäß einer Variante der Erfindung einstellenden sägezahnähnlichen Verlauf der Wechsel-Hochspannung, und
- Fig. 4: einen schematischen Schaltplan einer anderen Ausführungsform einer erfindungsgemäßen Vorrichtung.

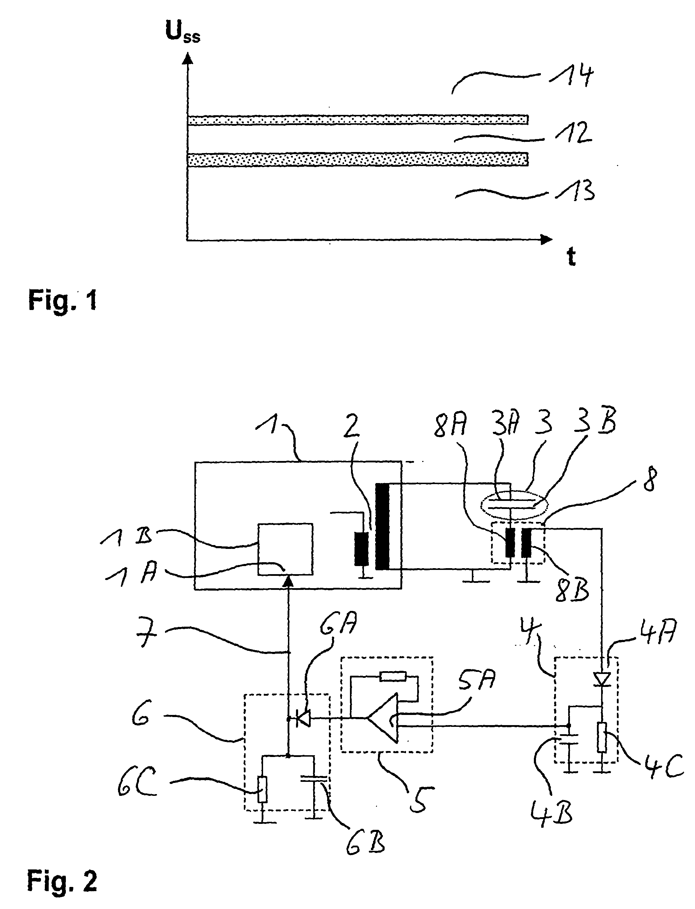

Figur 1 zeigt, daß bei Anschalten einer Wechsel-Hochspannung an z.B. ein planares Flachmodul nach der DE199 31 366.0 , vorzugsweise einer mit einer Frequenz von 20-100KHz, ab einer bestimmten, von der Geometrie abhängigen elektrischen Feldstärke es aufgrund des Auftretens von Feldüberhöhungen an den Elektroden zur Produktion von negativen und positiven Ionen kommt. Der zugehörige Bereich der Wechsel-Hochspannung ist in Fig. 1 mit dem Bezugszeichen 12 markiert.

Bei weiter anwachsender elektrischer Feldstärke beginnen elektrische Entladungen nach dem Prinzip der dielektrisch behinderten Entladung. Der zugehörige Bereich der Wechsel-Hochspannung ist in Fig. 1 mit dem Bezugszeichen 14 markiert. Die den Einzelentladungen, "Filamente" genannt, innewohnende kinetische Energie dissossiiert zweiatomige Sauerstoffmoleküle O₂ zu O₁, die sich zu O₃ rekombinieren. In dem mit dem Bezugszeichen 13 markierten Bereich reicht die Wechsel-Hochspannung auch zur Ionenproduktion nicht mehr aus.

Der Arbeitspunkt eines nach dem Prinzip der dielekrisch behinderten Entladung arbeitenden Gasentladungsmoduls bzw. Ionisationsgerätes kann so gewählt werden, daß eine maximale Ionisation erfolgt, und daß dabei eine so geringe Menge Ozon produziert wird, daß einerseits die sterilisierende und deodorierende Wirkung von Ozon genutzt wird, ohne daß dabei der Ozon riechbar wird oder Grenzwerte überschritten werden können.

Figur 2, welche einen schematischen Schaltplan einer Ausführungsform einer erfindungsgemäßen Vorrichtung zeigt, veranschaulicht beispielhaft die Funktionszusammenhänge der Erfindung.

In einem Hochspannungserzeuger 1 befindet sich eine Ansteuerungsschaltung 1B für den Hochspannungstransformator 2. Die Wechsel-Hochspannung gelangt über die Primärwicklung 8A eines Hochfrequenzübertragers 8 auf das Ionisations- und Ozonisierungsmodul bzw. Gasentladungsmodul 3, welches elektrisch gesehen eine Kapazität ist. Die elektrischen Daten des Hochfrequenzübertragers 8 sind so gewählt, daß die Grundfrequenz der Ansteuerhochspannung bzw. Wechsel-Hochspannung, welche zwischen 20-100KHz sein kann, nicht übertragen wird. Vielmehr werden Signale einer Frequenz von größer 1-2 MHz übertragen. Denn in diesem hohen Frequenzbereich liegen Signale, die typisch von dielektrisch behinderten Entladungsimpulsen ausgehen. Diese hochfrequenten Entladungsimpulse werden über die aus einer Diode 4A, einem Kondensator 4B und einem Widerstand 4C bestehende Demodulatorschaltung 4 gleichgerichtet und geglättet.

In einem Verstärker 5 wird das so gewonnene Signal verstärkt und auf einen Integrierer 6 gegeben. Die so gewonnene Regelspannung wird wieder dem Hochspannungserzeuger 1 zugeführt, indem die Regelspannung über eine Zuleitung 7 auf den Steuereingang 1A der Ansteuerungsschaltung 1B aufgegeben wird.

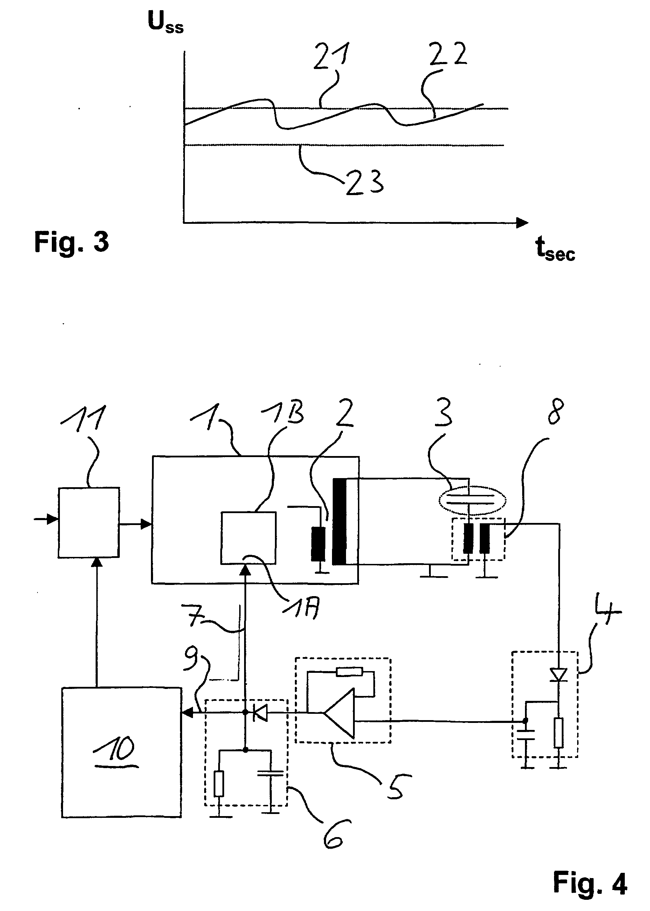

Das Gasentladungsmodul 3 ist imstande, Ionen und Ozon in der Luft zu erzeugen, und weist zwei Elektroden 3A, 3B auf, zwischen denen die vom Hochspannungserzeuger 1 erzeugte eine Wechsel-Hochspannung und somit ein elektrisches Feld anliegt. Es ist ein bestimmter Schwellenwert der Wechsel-Hochspannung gegeben, oberhalb desselben durch das Gasentladungsmodul 3 elektrische Entladeimpulse, welche ihrerseits Ozon produzieren, und Ionen erzeugt werden. Dieser Schwellenwert liegt in Fig. 1 zwischen den mit den Bezugszeichen 14 und 12 markierten Spannungsbereichen und ist in Fig. 3 mit dem Bezugszeichen 21 markiert.

Durch die Entladeimpulse werden die erwähnten schnellen elektrischen Signale hervorgerufen. Diese werden erfindungsgemäß zum Zwecke der automatischen Beschränkung der Ozonproduktion dazu herangezogen, mittels einer Rückkopplung die Wechsel-Hochspannung HV unter den Schwellenwert auf einen solchen Wert abzusenken, daß keine Entladeimpulse und somit kein Ozon mehr entstehen, jedoch weiterhin Ionen in dem elektrischen Feld erzeugt werden.

Die Wechsel-Hochspannung steigt erfindungsgemäß nach der Absenkung unter den Schwellenwert 21 wieder so weit über den Schwellenwert 21, daß erneut Entladeimpulse und somit Ozon erzeugt werden, welche die Wechsel-Hochspannung wiederum unter den Schwellenwert 21 absenken und so fort, so dass aufgrund der Rückkopplung die Wechsel-Hochspannung um den Schwellenwert 21 pendelt (Fig. 3). Gemäß einer bevorzugten Variante erfolgt die Absenkung der Hochspannung mit einer betragsmäßig höheren zeitlichen Änderungsrate als die Erhöhung der Wechsel-Hochspannung, so daß sich ein jeweils sägezahnähnlicher zeitlicher Verlauf der Hochspannung ergibt; ein Beispiel für einen derartigen Verlauf ist in Fig. 3 mit dem Bezugszeichen 22 markiert.

Der Signalweg der Rückkopplung nach dem Gasentladungsmodul 3 umfaßt in der Vorrichtung von Fig. 2 den Hochfrequenzübertrager 8, den Demodulator 4, den Verstärker 5 mit einem Eingang 5A sowie den Integrierer 6. Der Hochspannungserzeuger 1 umfaßt eine Ansteuerungsschaltung 1B, welche einen Steuereingang 1A besitzt.

Der Hochfrequenzübertrager 8 ist vorzugsweise so eingerichtet, daß er die Grundfrequenz der Wechsel-Hochspannung, die typischweise bei 20...1200 kHz liegt, im wesentlichen nicht überträgt, so daß der Hochfrequenzübertrager 8 die erwähnten Signale im wesentlichen von der Wechsel-Hochspannung abtrennt.

Der Demodulator 4 umfaßt eine erste Diode 4A, einen ersten Kondensator 4B und einen ersten Widerstand 4C umfaßt, wobei die erste Diode 4A zum Zweck der Gleichrichtung der elektrischen Signale zwischen den Hochfrequenz-Übertrager 8 und den Verstärker 5 zwischengeschaltet ist, und der Eingang 5A des Verstärkers 5 über den ersten Kondensator 4B und parallel hierzu über den ersten Widerstand 4C an Masse gelegt ist. Der Verstärker 5 kann z.B. ein Impedanzwandler sein. Der Integrierer 6 umfaßt eine zweite Diode 6A, einen zweiten Kondensator 6B und einen zweiten Widerstand 6C, wobei die zweite Diode 6A zwischen den Verstärker 5 und den Steuereingang 1A zwischengeschaltet ist, und der Steuereingang 1A über die Zuleitung 7 und den zweiten Kondensator 6B und parallel zu diesem über den zweiten Widerstand 6C an Masse gelegt ist.

Die Ansteuerschaltung 1B ist imstande, die Wechsel-Hochspannung so zu steuern, daß diese konstant bleibt, falls am Steuereingang 1A eine konstante Spannung anliegt, ansteigt, falls am Steuereingang 1A eine abnehmende Spannung anliegt, und absinkt, falls am Steuereingang 1A eine zunehmende Spannung anliegt. Die Größe der Wechsel-Hochspannung verhält sich daher gegenläufig zur Größe der am Steuereingang 1A anliegenden Spannung. Die Primärwicklung 8A des Hochfrequenzübertragers 8 ist mit dem Gasentladungsmodul 3 in Serie geschaltet und überträgt die elektrischen Signale induktiv auf die Sekundärwicklung 8B, welche die Signale an den Demodulator 4 abgibt. Die Sekundärwicklung 8B ist einseitig an Masse gelegt. Der Demodulator 4 richtet die Signale gleich und glättet sie und gibt sie danach als Eingangssignal an den Eingang 5A des Verstärkers 5 ab. Der Verstärker 5 verstärkt das Eingangssignal z.B. strommäßig gibt das verstärkte Signal als Ausgangssignal an den Integrierer 6 abgibt. Der Integrierer 6 integriert das Ausgangssignal mit einer vorgegebenen Zeitkonstanten zu einem Regelsignal und gibt dieses dem Steuereingang 1A auf.

Damit ergibt sich die Funktion, daß bei Auftreten dielektrisch behinderter Entladungen eine Regelspannung erzeugt wird, welche die Amplitude der Ansteuerhochspannung bzw. Wechsel-Hochspannung verringert. Somit verlöschen die dieelektrisch behinderten Entladungen wieder. Die Entladung des Integrieres 6 erfolgt nach einer langsameren Zeitkonstante als die Aufladung des Integrierers 6, welche schneller erfolgt. Damit ergibt sich eine Funktion wie in Figur 3 beschrieben: Oberhalb der durch die Linie 21 gekennzeichneten Spannung, Schwellenwert 21, zünden Entladungen, welche aufgrund der diesen Entladungen innewohnenden kinetischen Energie Ozon zu produzieren in der Lage sind und die erwähnten schnellen elektrischen Signale hervorrufen. Unterhalb der Linie 21 bis zur Linie 23 bilden sich hohe elektrische Felder an den Elektroden der Ionisations-und Ozonisationsapparate bzw. des Gasentladungsmoduls 3 aus, welche die Luft und luftgetragene Partikel in der Umgebung ionisiert.

Der Einsatz der in Figur 2 vorgestellten Schaltung läßt die Ansteuerspannung stets um die Linie 21 oszillieren bzw. pendeln. Es ist damit sichergestellt, daß einerseits niemals in bedeutendem Umfang Ozon erzeugt wird, zum anderen ist langzeitstabil die immer gleiche Ionenproduktion gewährleistet, unabhängig von Bauteiletoleranzen, Spannungsschwankungen, Schwankungen der Temperatur oder der Luftfeuchte und weiterer Randparameter.

Im Ergebnis kann in manchen Anwendungen auf den der Ionisations- und Ozonisierungsstufe nachgeschalteten Katalysator verzichtet werden. Dies ist in Anwendungen im Kraftfahrzeug oder in sonstigen Klimatisierunganlagen vorteilhaft, weil die Sterilisierung des feuchten Verdampfers durch Beströmen mit hochionisierter Luft mit geringem, der Natur entsprechendem Ozonanteil von 20-40ppb sichergestellt ist und überschießende Ozonproduktion erfindungsgemäß nicht möglich ist.

Vorteilhaft gegenüber den bekannten Sprühelektroden nach dem Koronar-Prinzip ist die sehr lange Lebensdauer und die Datenkonstanz der nach dem Prinzip der dielektrisch behinderten Entladung arbeitenden planaren Flachmodule.

Die hier vorgestellte Erfindung macht sich die Tatsache zu Nutze, daß beim Anlegen einer hohen und vorzugsweise hochfrequenten Spannung an eine nach dem Prinzip der dielektrisch behinderten Entladung arbeitende Baugruppe, z.B. Siemensröhre oder planare Flachbaugruppe, entsprechend Fig. 1 erst bei Überschreiten einer bestimmten Spannung es zu Spontanentladungen, Filamenten, kommt, welche letztlich Ozon erzeugen. Im darunter liegenden Spannungsbereich 12 wird Luft ionisiert und die Ozonproduktion ist sehr gering. Würde man eine Betriebsspannung manuell einstellen, welche sich kurz unter oder im Beginn der dielektrischen Entladung befindet, könnte der so gewählte Arbeitsbereich durch Toleranzverschiebungen, Spannungsschwankungen etc. nach oben oder unten verschoben werden, was die Funktionen der Anordnung verschlechtert oder daß sogar unerwünschte oder gefährliche Ozonkonzentrationen auftreten.

Fig. 4 zeigt einen schematischen Schaltplan einer anderen Ausführungsform einer erfindungsgemäßen Vorrichtung. Die Vorrichtung von Fig. 4 weist gegenüber derjenigen von Fig. 2 zusätzlich eine Auswerteschaltung 10 sowie ein Schaltgerät 11 auf. Hierdurch ist die Schaltung von Figur 2 dahingehend ergänzt worden, daß eine unabhängige und übergeordnete Überwachung der Schaltungsfunktion erfolgt. Den Schaltungen von Fig. 2 und Fig. 4 ist zueigen, daß die Regelspannung nicht konstant ist, sondern um einen bestimmten Wert herum oszilliert, insofern also eine Dynamik aufweist.

Diese Spannung, Regelspannung, wird über eine Leitung 9 zusätzlich auf die elektrische Auswerteschaltung 10 gegeben, die nach dem Prinzip eines monostabilen Multivibrators arbeitet. Nach dem Einschalten wird der Ausgang der Auswerteschaltung 10 durch einen Startimpuls so gesetzt, daß dessen auf ein Schaltgerät 11 wirkender Ausgang die Versorgungsspannung des Hochspannungserzeugers 1 einschaltet. Die Auswerteschaltung 10 fällt automatisch nach kurzer Zeit in einen anderen logischen Zustand, wenn über die Leitung 9 keine Triggerimpulse den zuerst erreichten Zustand aufrechterhalten. Sollte also in irgendeiner Form der Regelkreis ausfallen, gebildet aus: Hochspannungserzeuger 1, Ionsationsmodul bzw. Gasentladungsmodul 3, Hochfrequenzübertrager 8, Demodulator 4, Verstärker 5 und Integrierer 6, dann würde die Regelspannung keine Dynamik mehr aufweisen, was die Auswerteschaltung 10 in den normalen Zustand kippen läßt mit der Folge, daß das Schaltgerät 11 sperrt und daß damit unabhängig und übergeordnet die Funktion des Hochspannungserzeugers 1 unterbrochen wird.

Vorteilhaft wird dadurch erreicht, daß ein so betriebenes Ionisations- und Ozonisierungsgerät nie unkontrolliert in einen gefährlichen Zustand gelangen kann, indem unkontrolliert Ozon in unzuträglichen Mengen produziert werden könnte. Vorteilhaft ist weiter, daß selbst bei langzeitbedingter Drift technischer Daten einzelner Komponenten niemals Ozon produziert werden könnte, weil die dem geschlossenen Regelkreis innewohnende Logik dies sicher verhindert. Dies ist insbesondere dann wichtig, wenn die von Luftaufbereitungs- und Klimatisationsgeräten behandelte Luft in kleine Räume abgegebenen wird, in denen sich Ozon im Laufe der Zeit anreichern könnte.

Die hier vorgestellte Funktion kann in zahlreichen technischen Varianten, abweichend vom gewählten Schaltungsvorschlag realisiert werden. Die Funktion kann auch in einer digitalisierten, mikroprozessorgestützten Version realisiert werden. Immer ist jedoch der erfindungsgemäße Gedanke zielführend, daß die Wechsel-Hochspannung durch einen geschlossenen Regelkreis in einen Bereich gebracht wird, welcher den Betrieb der Ionisations- und Ozonisationsbaugruppe automatisch in einem Band hält, in welchem eine maximale und sichere Ionisierung bei gleichzeitig geringer Ozonproduktion erfolgt.

Der vorteilhafte Einbau von Baugruppen im Sinne der Erfindung kann überall dort erfolgen, wo das freie Ausblasen von Ozon in von Menschen frequentierten Räumen sicher vermieden werden soll und ein den Ozon abbauender Katalysator technisch nicht möglich ist oder nicht erwünscht ist. Dies kann insbesondere in Klimatisierungs- und Luftkonditionierungsanlagen aller Art der Fall sein, insbesondere jedoch dort, wo auf der feuchten Oberfläche eines die Luft kühlenden Wärmetauschers Keime und Pilze aller Art sicher verhindert werden sollen.

### Gewerbliche Anwendbarkeit:

Die Erfindung ist gewerblich anwendbar z.B. im Bereich der Luftaufbereitung mit Ozon sowie der Klimatechnik insbesondere in Kraftfahrzeugen oder in Krankenhäusern.

### Liste der Bezugszeichen:

- 1: Hochspannungserzeuger
- 1A: Eingang von 1B
- 1B: Ansteuerungsschaltung
- 2: Hochspannungstransformator
- 3: Gasentladungsmodul
- 3A,3B: Elektroden von 3
- 4: Demodulator
- 4A,4B,4C: erste Diode, erste Kondensator, erster Widerstand
- 5: Verstärker
- 6: Integrierer
- 6A,6B,6C: zweite Diode, zweiter Kondensator, zweiter Widerstand
- 7: Zuleitung für Regelsignal
- 8: Hochfrequenzübertrager
- 9: Leitung
- 8A,8B: Primärwicklung, Sekundärwicklung von 8
- 10: Auswerteschaltung
- 11: Schaltgerät
- 12,13,14: Spannungsbereiche
- 21: Schwellenwert
- 22: zeitlicher Verlauf der Wechsel-Hochspannung
- 23: Spannungswert

## Patentansprüche

1. Verfahren zum Betreiben eines elektrischen Gasentladungsmoduls (3), insbesondere für dielektrisch behinderte Entladung, wobei das Gasentladungsmodul (3) imstande ist, Ionen und Ozon in der Luft zu erzeugen und zwei Elektroden (3A, 3B) aufweist, zwischen denen eine Wechsel-Hochspannung aus einem Hochspannungserzeuger (1) angelegt und somit ein elektrisches Feld erzeugt wird, **dadurch gekennzeichnet,**
**daß** ein bestimmter Schwellenwert (21) der Wechsel-Hochspannung gegeben ist, oberhalb desselben mittels des Gasentladungsmoduls (3) elektrische Entladeimpulse, welche ihrerseits Ozon produzieren, und Ionen erzeugt werden, wobei durch die Entladeimpulse elektrische Signale hervorgerufen werden, welche zum Zwecke der automatischen Beschränkung der Ozonproduktion mittels einer Rückkopplung dazu herangezogen werden, die Wechsel-Hochspannung unter den Schwellenwert (21) auf einen solchen Wert abzusenken, daß keine Entladeimpulse und somit kein Ozon mehr entstehen, jedoch weiterhin Ionen in dem elektrischen Feld erzeugt werden.

2. Verfahren nach Anspruch 1, **dadurch gekennzeichnet,**
**daß** die Wechsel-Hochspannung nach der Absenkung unter den Schwellenwert (21) wieder so weit über den Schwellenwert (21) erhöht wird, daß erneut Entladeimpulse und somit Ozon erzeugt werden, welche wiederum dazu herangezogen werden, die Wechsel-Hochspannung wiederum unter den Schwellenwert (21) abzusenken und so fort, so dass aufgrund der Rückkopplung die Wechsel-Hochspannung um den Schwellenwert pendelt.

3. Verfahren nach Anspruch 1 oder 2, **dadurch gekennzeichnet,**
**daß** die durch einen Hochspannungserzeuger (1) mit einem Steuereingang (1A) erzeugte Wechsel-Hochspannung bei Anlegen einer anwachsenden Spannung an den Steuereingang (1A) abnimmt und umgekehrt, wobei aus den elektrischen Signalen eine Regelspannung gewonnen wird, welche an den Steuereingang (1A) rückgekoppelt wird.

4. Verfahren nach Anspruch 3, **dadurch gekennzeichnet,**
**dass** zur Gewinnung der Regelspannung aus den Signalen dieselben von der Hochspannung abgekoppelt, gleichgerichtet und geglättet werden, wobei gegebenenfalls die Signale nach der Glättung zusätzlich verstärkt und integriert werden.

5. Verfahren nach Anspruch 1 oder 2, **dadurch gekennzeichnet,**
**daß** die Absenkung oder die Erhöhung der Wechsel-Hochspannung treppenfunktionsartig erfolgt.

6. Verfahren nach Anspruch 2, **dadurch gekennzeichnet,**
**daß** die Absenkung der Wechsel-Hochspannung mit einer betragsmäßig höheren zeitlichen Änderungsrate erfolgt als die Erhöhung der Hochspannung, so daß sich ein jeweils sägezahnähnlicher zeitlicher Verlauf der Regelspannung sowie der Hochspannung ergibt.

7. Verfahren nach einem der Ansprüche 1 oder 2, **dadurch gekennzeichnet,**
**daß** die Wechsel-Hochspannung um einen vorgegebenen Verringerungsbetrag abgesenkt oder um einen vorgegebenen Erhöhungsbetrag erhöht wird.

8. Verfahren nach einem der Ansprüche 1 oder 2, **dadurch gekennzeichnet,**
**daß** die Wechsel-Hochspannung auf eine vorgegebene, unterhalb der Schwellenspannung liegende Minimalspannung abgesenkt oder auf eine vorgegebene, oberhalb der Schwellenspannung 21 liegende Maximalspannung erhöht wird.

9. Verfahren nach einem der Ansprüche 3 oder 4,
**dadurch gekennzeichnet, daß** die Absenkung und die Erhöhung der Wechsel-Hochspannung mittels eines Mikroprozessors erfolgen, an welchen die Regelspannung angelegt wird.

10. Verfahren nach einem der Ansprüche 1 bis 9, **dadurch gekennzeichnet,**
**daß** die Wechsel-Hochspannung (HV) durch eine Überwachungsschaltung (10,11), welche das zeitliche Verhalten der Regelspannung überwacht, dann abgeschaltet wird, wenn die Dynamik der Regelspannung für mindestens eine vorgegebene Kontrollzeit eine vorgegebene Mindest-Dynamik nicht erreicht.

11. Verfahren nach Anspruch 1 oder 10, **dadurch gekennzeichnet,**
**dass** die um einen bestimmten Wert pendelnde Regelspannung (7) über eine Leitung (9) als Triggerimpulse auf eine elektrische Auswerteschaltung (10) gegeben wird, welche nach dem Prinzip eines monostabilen Multivibrators arbeitet und einen Ausgang aufweist, wobei mit Beginn des Verfahrens der Ausgang der Auswerteschaltung (10) durch einen Startimpuls so gesetzt wird, daß dessen auf ein Schaltgerät (11) wirkender Ausgang die Versorgungsspannung des Hochspannungserzeugers (1) einschaltet, wobei, falls über die Leitung (9) keine Triggerimpulse den zuerst erreichten Zustand aufrechterhalten, die Auswerteschaltung (10) automatisch in den anderen logischen Zustand fällt, und dadurch das Schaltgerät (11) die Funktion des Hochspannungserzeugers (1) in übergeordneter Funktion unterbricht.

12. Vorrichtung zum Betreiben eines elektrischen Gasentladungsmoduls (3), insbesondere für dielektrisch behinderte Entladung, wobei das Gasentladungsmodul (3) imstande ist, Ionen und Ozon in der Luft zu erzeugen und zwei Elektroden (3A, 3B) aufweist, zwischen denen aus einem Hochspannungserzeuger (1) eine Wechsel-Hochspannung und somit ein elektrisches Feld anliegt, **dadurch gekennzeichnet,**
**daß** ein bestimmter Schwellenwert (21) der Wechsel-Hochspannung gegeben ist, oberhalb desselben durch das Gasentladungsmodul (3) elektrische Entladeimpulse, welche ihrerseits Ozon produzieren, und Ionen erzeugt werden, wobei durch die Entladeimpulse elektrische Signale hervorgerufen werden, welche zum Zwecke der automatischen Beschränkung der Ozonproduktion dazu dienen, mittels einer Rückkopplung die Wechsel-Hochspannung unter den Schwellenwert (21) auf einen solchen Wert abzusenken, daß keine Entladeimpulse und somit kein Ozon mehr entstehen, jedoch weiterhin Ionen in dem elektrischen Feld erzeugt werden.

13. Vorrichtung nach Anspruch 12, **dadurch gekennzeichnet,**
**dass** die Wechsel-Hochspannung nach der Absenkung unter den Schwellenwert (21) wieder so weit über den Schwellenwert steigt, daß erneut Entladeimpulse und somit Ozon erzeugt werden, welche die Wechsel-Hochspannung wiederum unter den Schwellenwert (21) absenken und so fort, so dass aufgrund der Rückkopplung die Wechsel-Hochspannung um den Schwellenwert (21) pendelt.

14. Vorrichtung nach Anspruch 13, **dadurch gekennzeichnet,**
**daß** der Signalweg der Rückkopplung nach dem Gasentladungsmodul (3) einen Hochfrequenzübertrager (8), einen Demodulator (4), einen Verstärker (5) mit einem Eingang (5A) sowie einen Integrierer (6) aufweist und der Hochspannungserzeuger (1) eine Ansteuerungsschaltung (1B) umfaßt, wobei
- die Ansteuerungsschaltung (1B) einen Steuereingang (1A) aufweist und die Wechsel-Hochspannung so zu steuern imstande ist, daß diese
- konstant bleibt, falls am Steuereingang (1A) eine konstante Spannung anliegt,
- ansteigt, falls am Steuereingang (1A) eine abnehmende Spannung anliegt, und
- absinkt, falls am Steuereingang (1A) eine zunehmende Spannung anliegt, so daß sich die Größe der Wechsel-Hochspannung gegenläufig zur Größe der am Steuereingang (1A) anliegenden Spannung verhält,
- die Primärwicklung (8A) des Hochfrequenzübertragers (8) mit dem Gasentladungsmodul (3) in Serie geschaltet ist und die elektrischen Signale induktiv auf die Sekundärwicklung (8B) überträgt, welche die Signale an den Demodulator (4) abgibt und welche einseitig an Masse gelegt ist,
- der Demodulator (4) die Signale gleichrichtet und glättet und danach als Eingangssignal an den Eingang (5A) des Verstärkers abgibt,
- der Verstärker (5) das Eingangssignal verstärkt und als Ausgangssignal an den Integrierer (6) abgibt, und
- der Integrierer (6) das Ausgangssignal mit einer vorgegebenen Zeitkonstanten zu einem Regelsignal (7) integriert und dieses dem Steuereingang (1A) aufgibt.

15. Vorrichtung nach Anspruch 14, **dadurch gekennzeichnet,**
**daß** der Hochfrequenzübertrager (8) die Grundfrequenz der Wechsel-Hochspannung im wesentlichen nicht überträgt, so daß der Hochfrequenzübertrager (8) die Signale im wesentlichen von der Wechsel-Hochspannung abtrennt.

16. Vorrichtung nach Anspruch 14 oder 15, **dadurch gekennzeichnet,**
**daß** der Demodulator (4) eine erste Diode (4A), einen ersten Kondensator (4B) und einen ersten Widerstand (4C) umfaßt, wobei
- die erste Diode (4A) zum Zweck der Gleichrichtung der elektrischen Signale zwischen den Hochfrequenz-Übertrager (8) und den Verstärker (5) zwischengeschaltet ist, und
- der Eingang (5A) des Verstärkers (5) über den ersten Kondensator (4B) und parallel hierzu über den ersten Widerstand (4C) an Masse gelegt ist.

17. Vorrichtung nach einem der Ansprüche 14 bis 16, **dadurch gekennzeichnet, daß** der Verstärker (5) ein Impedanzwandler ist.

18. Vorrichtung nach einem der Ansprüche 14 bis 17, **dadurch gekennzeichnet,**
**daß** der Integrierer (6) eine zweite Diode (6A), einen zweiten Kondensator (6B) und einen zweiten Widerstand (6C) umfaßt, wobei
- die zweite Diode (6A) zwischen den Verstärker (5) und den Steuereingang (1A) zwischengeschaltet ist, und
- der Steuereingang (1A) über den zweiten Kondensator (6B) und parallel hierzu über den zweiten Widerstand (6C) an Masse gelegt ist.

19. Vorrichtung nach einem der Ansprüche 14 bis 18, **dadurch gekennzeichnet,**
**daß** der aus Hochspannungserzeuger (1), Gasentladungsmodul (3), Hochfrequenzübertrager (8), Demodulator (4), Verstärker (5) und Integrierer (6) gebildete Regelkreis eine große Regelungssteilheit und hohe Hysterese aufweist, wobei die Zeitkonstante für das Ansprechen der Regelschleife kleiner ist als die Zeitkonstante für das Abklingen der Regelspannung (7) mit dem Ergebnis, daß die Regelspannung (7) eine Sägezahnfunktion bildet.

20. Vorrichtung nach Anspruch 13, **dadurch gekennzeichnet,**
**daß** dieselbe einen Mikroprozessor aufweist, an welchen die Regelspannung angelegt ist und welcher die Wechsel-Hochspannung (HV) erhöht und absenkt.

21. Vorrichtung nach einem der Ansprüche 12 bis 20, **dadurch gekennzeichnet,**
**daß** dieselbe eine Überwachungsschaltung (10,11) umfaßt, welche das zeitliche Verhalten der Regelspannung überwacht und die Wechsel-Hochspannung (HV) dann abschaltet, wenn die Dynamik der Regelspannung für mindestens eine vorgegebene Kontrollzeit eine vorgegebene Mindest-Dynamik nicht erreicht.

## Claims

1. A method to operate an electric gas discharge module (3), especially for dielectrically hindered discharge, whereby the gas discharge module (3) is capable of generating ions and ozone in the air, and it has two electrodes (3A, 3B), between which an alternating high voltage from a high-voltage generator (1) is applied and thus an electric field is generated, **characterized in that**
a certain threshold value (21) exists for the alternating high voltage, above which electric discharge pulses ― which, in turn, produce ozone ― as well as ions are generated by means of the gas discharge module (3), whereby the discharge pulses give rise to electric signals which, for purposes of automatically limiting the ozone production, use back coupling in order to lower the alternating high voltage below the threshold value (21) to such a value that no discharge pulses and thus no more ozone are produced but so that ions continue to be generated in the electric field.

2. The method according to Claim 1, **characterized in that**,
after being lowered to below the threshold value (21), the alternating high voltage is raised again above the threshold value (21) to such an extent that once again discharge pulses and thus ozone are generated which, in turn, are used to once again lower the alternating high voltage below the threshold value (21) and so forth, so that, due to the back coupling, the alternating high voltage fluctuates around the threshold value.

3. The method according to Claim 1 or 2, **characterized in that**
the alternating high voltage generated by a high-voltage generator (1) with a control input (1A) decreases when an increasing voltage is applied to the control input (1A) and vice versa, whereby a control voltage is obtained from the electric signals and said control voltage is coupled back to the control input (1A).

4. The method according to Claim 3, **characterized in that**
in order to obtain the control voltage from the signals, said signals are uncoupled from the high voltage, rectified and smoothed, whereby optionally the signals are additionally amplified and integrated after the smoothing.

5. The method according to Claim 1 or 2, **characterized in that**
the lowering or raising of the alternating high voltage takes place as a step function.

6. The method according to Claim 2, **characterized in that**
the lowering of the alternating high voltage takes place at a higher rate of change over time than the raising of the high voltage, thus yielding a sawtooth course over time of the control voltage as well as of the high voltage in each case.

7. The method according to one of Claims 1 or 2, **characterized in that**
the alternating high voltage is lowered by a predefined decrease amount or raised by a predefined increase amount.

8. The method according to one of Claims 1 or 2, **characterized in that**
the alternating high voltage is lowered to a predefined minimum voltage that lies below the threshold voltage or else is increased to a predefined maximum voltage that lies above the threshold voltage 21.

9. The method according to one of Claims 3 or 4, **characterized in that**
the lowering and the raising of the alternating high voltage are carried out by means of a microprocessor to which the control voltage is applied.

10. The method according to one of Claims 1 to 9, **characterized in that**
the alternating high voltage (HV) is switched off by a monitoring circuit (10, 11) that monitors the behavior over time of the control voltage whenever the dynamic response of the control voltage does not reach a predefined minimum dynamic response at least for a predefined control time.

11. The method according to Claim 1 or 10, **characterized in that**
the control voltage (7) fluctuating around a certain value is sent via a line (9) as trigger pulses to an electric evaluation circuit (10) that functions according to the principle of a monostable multi-vibrator and that has one output, whereby at the beginning of the method, the output of the evaluation circuit (10) is set by a starting pulse in such a way that its output that acts on a switching device (11) switches on the supply voltage of the high-voltage generator (1) whereby, if no trigger pulses maintain the state that was first reached via the line (9), the evaluation circuit (10) automatically moves into the other logical state, as a result of which the switching device (11) interrupts the function of the high-voltage generator (1) in a superordinated function.

12. A device to operate an electric gas discharge module (3), especially for dielectrically hindered discharge, whereby the gas discharge module (3) is capable of generating ions and ozone in the air, and it has it has two electrodes (3A, 3B), between which an alternating high voltage from a high-voltage generator (1) and thus an electric field is present, **characterized in that**
a certain threshold value (21) exists for the alternating high voltage, above which electric discharge pulses ― which, in turn, produce ozone ― as well as ions are generated by means of the gas discharge module (3), whereby the discharge pulses give rise to electric signals which, for purposes of automatically limiting the ozone production, use back coupling in order to lower the alternating high voltage below the threshold value (21) to such a value that no discharge pulses and thus no more ozone are produced but so that ions continue to be generated in the electric field.

13. The device according to Claim 12, **characterized in that**
after dropping to below the threshold value (21), the alternating high voltage rises again above the threshold value to such an extent that once again discharge pulses and thus ozone are generated which, in turn, once again lower the alternating high voltage below the threshold value (21) and so forth, so that, due to the back coupling, the alternating high voltage fluctuates around the threshold value (21).

14. The device according to Claim 13, **characterized in that**
the signal path of the back coupling after the gas discharge module (3) comprises a high-frequency transmitter (8), a demodulator (4), an amplifier (5) with an input (5A) as well as an integrator (6) and the high-voltage generator (1) comprises a control circuit (1B), whereby
- the control circuit (1B) has a control input (1A) and is capable of controlling the alternating high voltage in such a way that it
- remains constant if a constant voltage is applied to the control input (1A),
- rises if a decreasing voltage is applied to the control input (1A),
- drops if an increasing voltage is applied to the control input (1A), so that the magnitude of the alternating high voltage evolves counter to the magnitude of the voltage applied to the control input (1A),
- the primary winding (8A) of the high-frequency transmitter (8) is connected in series with the gas discharge module (3) and transmits the electric signals inductively to the secondary winding (8B) which emits the signals to the demodulator (4) and which is connected on one side to the ground,
- the demodulator (4) rectifies and smoothes the signals and then sends them as an input signal to the input (5A) of the amplifier,
- the amplifier (5) amplifies the input signal and sends it as an output signal to the integrator (6), and
- the integrator (6) integrates the output signal with a predefined time constant to form a control signal (7) and forwards the latter to the control input (1A).

15. The device according to Claim 14, **characterized in that**
the high-frequency transmitter (8) essentially does not transmit the basic frequency of the alternating high voltage so that the high-frequency transmitter (8) essentially separates the signals from the alternating high voltage.

16. The device according to Claim 14 or 15, **characterized in that**
the demodulator (4) comprises a first diode (4A), a first capacitor (4B) and a first resistor (4C), whereby,
- for purposes of rectifying the electric signals, the first diode (4A) is connected between the high-frequency transmitter (8) and the amplifier (5), and
- the input (5A) of the amplifier (5) is connected to the ground via the first capacitor (48) and, parallel to this, via the first resistor (4C).

17. The device according to one of Claims 14 to 16, **characterized in that** the amplifier (5) is an impedance converter.

18. The device according to one of Claims 14 to 17, **characterized in that**
the integrator (6) comprises a second diode (6A), a second capacitor (6B) and a second resistor (6C), whereby
- the second diode (6A) is connected between the amplifier (5) and the control input (1A), and
- the control input (1A) is connected to the ground via the second capacitor (6B) and, parallel to this, via the second resistor (6C).

19. The device according to one of Claims 14 to 18, **characterized in that**
the control circuit consisting of the high-voltage generator (1), the gas discharge module (3), the high-frequency transmitter (8), the demodulator (4), the amplifier (5) and the integrator (6) has a high control transconductance and high hysteresis, whereby the time constant for the response of the control loop is smaller than the time constant for the decay of the control voltage (7), with the result that the control voltage (7) displays a sawtooth function.

20. The device according to Claim 13, **characterized in that**
said device has a microprocessor to which the control voltage is applied and which raises and lowers the alternating high voltage (HV).

21. The device according to one of Claims 12 to 20, **characterized in that**
said device comprises a monitoring circuit (10, 11) that monitors the behavior over time of the control voltage and that switches off the alternating high voltage (HV) whenever the dynamic response of the control voltage does not reach a predefined minimum dynamic response at least for a predefined control time.

## Revendications

1. Procédé de fonctionnement d'un module électrique à décharge gazeuse (3), en particulier pour décharge empêchée diélectriquement, le module à décharge gazeuse (3) étant en mesure de produire des ions et de l'ozone dans l'air et présentant deux électrodes (3A, 3B) entre lesquelles est appliquée une haute tension alternative provenant d'un générateur à haute tension (1) et un champ électrique étant ainsi produit, **caractérisé en ce que**
une certaine valeur de seuil (21) de la haute tension alternative est donnée, au-delà de laquelle à l'aide d'un module à décharge gazeuse (3) des impulsions de décharge électrique, lesquelles pour leur part produisent de l'ozone, et des ions sont générés, des impulsions de décharge provoquant des signaux électriques auxquels on a recours dans le but de limiter automatiquement la production d'ozone au moyen d'une rétroaction pour abaisser la haute tension alternative au-dessous de la valeur de seuil (21) à une valeur telle qu'aucune impulsion de décharge n'est produite et de ce fait plus d'ozone non plus, toutefois des ions continuant d'être générés dans le champ électrique.

2. Procédé selon la revendication 1, **caractérisé en ce que**
la haute tension alternative après l'abaissement au-dessous de la valeur de seuil (21) est à nouveau augmentée au-dessus de la valeur de seuil (21) dans une telle mesure que des impulsions de décharge et de ce fait de l'ozone sont à nouveau générés, impulsions de décharge auxquelles on a à nouveau recours pour abaisser à nouveau la haute tension alternative au-dessous de la valeur de seuil (21) et ainsi de suite, si bien qu'en raison de la rétroaction, la haute tension alternative oscille autour de la valeur de seuil.

3. Procédé selon l'une des revendications 1 ou 2, **caractérisé en ce que**
la haute tension alternative générée par le générateur de haute tension (1) comportant une entrée de commande (1A) diminue par l'application à l'entrée de commande (1A) d'une tension allant croissant et vice-versa, une tension de réglage étant obtenue à partir des signaux électriques, laquelle est rétroactivée à l'entrée de commande (1A).

4. Procédé selon la revendication 3, **caractérisé en ce que**,
pour l'obtention de la tension de réglage à partir des signaux, ceux-ci sont désaccouplés de la haute tension, redressés et égalisés, les signaux étant le cas échéant additionnellement amplifiés et intégrés.

5. Procédé selon l'une des revendications 1 ou 2, **caractérisé en ce que**
la baisse ou la hausse de la haute tension alternative se produit à la manière d'une fonction échelonnée.

6. Procédé selon la revendication 2, **caractérisé en ce que**,
la baisse de la haute tension alternative a lieu avec un taux de modification dont la grandeur dans le temps est plus élevée que la hausse de la haute tension, si bien qu'il en résulte une courbe dans le temps en dents de scie respectivement pour la tension de réglage, ainsi que pour la haute tension.

7. Procédé selon l'une des revendications 1 ou 2, **caractérisé en ce que**
la haute tension alternative est abaissée d'une grandeur de réduction déterminée ou augmentée d'une grandeur de hausse déterminée.

8. Procédé selon l'une des revendications 1 ou 2, **caractérisé en ce que**
la haute tension alternative est abaissée à une tension minima déterminée qui se situe au-dessous de la tension de seuil ou est augmentée à une tension maxima déterminée qui se situe au-delà de la tension de seuil 21.

9. Procédé selon l'une des revendications 3 ou 4, **caractérisé en ce que**
la baisse et la hausse de la haute tension alternative s'effectuent au moyen d'un microprocesseur auquel la tension de réglage est appliquée.

10. Procédé selon l'une ou l'autre des revendications 1 à 9,
**caractérisé en ce que**
la haute tension alternative (HV) est coupée par un circuit de contrôle (10, 11), lequel surveille le comportemet de la tension de réglage dans le temps, lorsque la dynamique de la tension de réglage n'atteint pas pendant tout au moins un temps de contrôle déterminé une dynamique minima définie.

11. Procédé selon l'une des revendications 1 ou 10, **caractérisé en ce que**
la tension de réglage (7) oscillant autour d'une certaine valeur est donnée via une ligne (9) en tant qu'impulsion de déclenchement sur un circuit électrique d'évaluation (10), lequel travaille d'après le principe d'un multivibrateur monostable et présente une sortie, la sortie du circuit d'analyse (10) étant réglée, dès le début du procédé, par une impulsion de départ de telle façon que sa sortie qui agit sur un appareil de commande (11) met en circuit la tension d'alimentation du générateur de haute tension (1), cela dit, au cas où aucune impulsion de déclenchement via la ligne (9) ne maintienne l'état atteint en premier, le circuit d'évaluation (10) tombe automatiquement dans l'autre état logique, et de ce fait, le circuit de commande (11) interrompt la fonction du générateur de haute tension (1) en tant que fonction prioritaire.

12. Dispositif de fonctionnement d'un module électrique à décharge gazeuse (3), en particulier pour décharge empêchée diélectriquement, le module à décharge gazeuse (3) étant en mesure de produire des ions et de l'ozone dans l'air et présentant deux électrodes (3A, 3B) entre lesquelles est appliquée une haute tension alternative provenant d'un générateur à haute tension (1) et ainsi un champ électrique, **caractérisé en ce que**
une certaine valeur de seuil (21) de la haute tension alternative est donnée, au-delà de laquelle au moyen du module à décharge gazeuse (3) des impulsions de décharge électrique, lesquelles pour leur part produisent de l'ozone, et des ions sont générés, des impulsions de décharge provoquant des signaux électriques, lesquels, dans le but de limiter automatiquement la production d'ozone au moyen d'une rétroaction, servent à abaisser la haute tension alternative au-dessous de la valeur de seuil (21) à une valeur telle que plus aucune impulsion de décharge n'est produite et de ce fait plus d'ozone non plus, toutefois des ions continuant d'être générés dans le champ électrique.

13. Dispositif selon la revendication 12, **caractérisé en ce que**
la haute tension alternative après l'abaissement au-dessous de la valeur de seuil (21) est à nouveau augmentée au-dessus de la valeur de seuil (21) dans une telle mesure que des impulsions de décharge et de ce fait de l'ozone sont à nouveau générés, lesquelles abaissent à nouveau la haute tension alternative au-dessous de la valeur de seuil (21) et ainsi de suite, de sorte que, en raison de la rétroaction, la haute tension alternative oscille autour de la valeur de seuil (21).

14. Dispositif selon la revendication 13, **caractérisé en ce que**
le chemin de signal de la rétroaction en aval du module à décharge gazeuse (3) présente un transformateur haute tension (8), un démodulateur (4), un amplificateur (5) avec une entrée (5A), ainsi qu'un intégrateur (6) et le générateur de haute tension (1) comprend un circuit d'amorçage (1B),
- le circuit d'amorçage (1B) présentant une entrée de commande (1A) et étant en mesure de commander la haute tension alternative de manière à ce que celle-ci
- reste constante au cas où une tension constante est appliquée à l'entrée de commande (1A),
- augmente au cas où une tension décroissante est appliquée à l'entrée de commande (1A) et
- diminue, au cas où une tension croissante est appliquée à l'entrée de commande (1A),
de sorte que la grandeur de la haute tension alternative se comporte de façon inverse à la grandeur de la tension appliquée à l'entrée de commande (1A),
- l'enroulement du circuit primaire (8A) du transformateur haute fréquence (8) est couplé en série avec le module à décharge gazeuse (3) et transmet les signaux électriques par induction à l'enroulement du circuit secondaire (8B) lequel transmet les signaux au démodulateur (4) et lequel est mis à la terre d'un seul côté.
- le démodulateur (4) redresse les signaux et les égalise et les transmet ensuite en tant que signal d'entrée à l'entrée (5A) de l'amplificateur,
- l'amplificateur (5) amplifie le signal d'entrée et le transmet en tant que signal de sortie à l'intégrateur (6), et
- l'intégrateur (6) intègre le signal de sortie avec une constante dans le temps déterminée en un signal de régulation (7) et transmet celui-ci à l'entrée de commande (1A).

15. Dispositif selon la revendication 14, **caractérisé en ce que**
le transformateur haute fréquence (8) ne transmet pas essentiellement la fréquence de base de la haute tension alternative, si bien que le transformateur haute fréquence (8) coupe les signaux essentiellement de la haute tension alternative.

16. Dispositif selon l'une des revendications 14 ou 15, **caractérisé en ce que**
le démodulateur (4) comprend une première diode (4A), un premier condensateur (4B) et une première résistance (4C),
- la première diode (4A) étant interconnectée dans le but de redresser les signaux électriques entre le transformateur haute fréquence (8) et l'amplificateur (5) et
- l'entrée (5A) de l'amplificateur (5) est mise à la masse via le premier condensateur (4B) et parallèlement à cela via la première résistance (4C).

17. Dispositif selon l'une des revendications 14 à 16, **caractérisé en ce que** l'amplificateur (5) est un transformateur d'adaptation d'impédance.

18. Dispositif selon l'une des revendications 14 à 17, **caractérisé en ce que**
l'intégrateur (6) comprend une deuxième diode (6A), un deuxième condensateur (6B) et une deuxième résistance (6C),
- la deuxième diode (6A) étant interconnectée entre l'amplificateur (5) et l'entrée de commande (1A) et
- l'entrée de commande (1A) est mise à la masse via le premier condensateur (4B) et parallèlement à cela via la deuxième résistance (6C).

19. Dispositif selon l'une des revendications 14 à 18, **caractérisé en ce que**
le circuit de réglage formé par le régulateur de haute tension (1), le module à décharge gazeuse (3), le transformateur haute fréquence (8), le démodulateur (4) l'amplificateur (5) et l'intégrateur (6) présente une grande pente de réglage et une hystérésis élevée, la constante de temps pour la réponse de la boucle de réglage étant plus petite que la constante de temps pour la décroissance de la tension de réglage (7) avec pour résultat que la tension de réglage (7) forme une fonction en dents de scie.

20. Dispositif selon la revendication 13, **caractérisé en ce que**
ledit dispositif présente un microprocesseur auquel est appliquée la tension de réglage et lequel augmente et abaisse la haute tension alternative (HV).

21. Dispositif selon l'une des revendications 12 à 20, **caractérisé en ce que**
ledit dispositif comprend un circuit de surveillance (10, 11), lequel surveille le comportement dans le temps de la tension de réglage et coupe la haute tension alternative (HV) lorsque la dynamique de la tension de réglage, pour tout au moins un temps de contrôle déterminé, n'atteint pas une dynamique minima prédéterminée.
